# EUROPEAN PATENT APPLICATION

(11) **EP 3 834 636 A1**
(43) Date of publication of application: **16.06.2021**
(21) Application number: 20168938.7
(22) Date of filing: 09.04.2020
(51) Int. Cl.: A24F 40/05, A24F 40/44, A61M 15/00, A61M 11/00, A61M 15/06, A61M 16/00

(54) **AN ULTRASONIC MIST INHALER DEVICE**

(30) Priority: 15.12.2019 WO PCT/IB2019/060808; 15.12.2019 WO PCT/IB2019/060809
(71) Applicant: Shaheen Innovations Holding Limited, Abu Dhabi (AE)
(72) Inventor: ALSHAIBA SALEH GHANNAM ALMAZROUEI, Mohammed, Abu Dhabi (AE); LAHOUD, Imad, Abu Dhabi (AE); BHATTI, Sajid, Abu Dhabi (AE)
(74) Representative: Forresters IP LLP

(57) **Abstract**

An ultrasonic mist inhaler device (300) comprising: a sonication chamber (301); an ultrasonic transducer (302) having an atomization surface (303) which is provided within the sonication chamber (301), the ultrasonic transducer (302) being configured to vibrate the atomization surface (303) to atomize a liquid within the sonication chamber (301); an airflow delivery member (313) which is coupled to the sonication chamber (301), the airflow delivery member (313) being configured to deliver air into the sonication chamber (301) along an airflow axis (314) which intersects the atomization surface (303); and a mist outlet arrangement (306) which provides a mist flow path (307) along which a mist of air and atomized liquid can flow out from the sonication chamber (301) to the exterior of the device (300).

## Description

### FIELD

The present invention relates to an ultrasonic mist inhaler device. The present invention more particularly relates to an ultrasonic mist inhaler device for atomizing a liquid by ultrasonic vibrations to produce a mist which is inhaled by a user.

### BACKGROUND

Electronic vaporizing inhalers are becoming popular among smokers who also want to avoid the tar and other harsh chemicals associated with traditional cigarettes and who wish to satisfy the craving for nicotine. Electronic vaporizing inhalers may contain liquid nicotine, which is typically a mixture of nicotine oil, a solvent, water, and sometimes flavoring. When the user draws, or inhales, on the electronic vaporizing inhaler, the liquid nicotine is drawn into a vaporizer where it is heated into a vapor. As the user draws on the electronic vaporizing inhaler, the vapor containing the nicotine is inhaled. Such electronic vaporizing inhalers may have medical purpose.

Electronic vaporizing inhalers and other vapor inhalers typically have similar designs. Most electronic vaporizing inhalers feature a liquid nicotine reservoir with an interior membrane, such as a capillary element, typically cotton, that holds the liquid nicotine so as to prevent leaking from the reservoir. Nevertheless, these cigarettes are still prone to leaking because there is no obstacle to prevent the liquid from flowing out of the membrane and into the mouthpiece. A leaking electronic vaporizing inhaler is problematic for several reasons. As a first disadvantage, the liquid can leak into the electronic components, which can cause serious damage to the device. As a second disadvantage, the liquid can leak into the electronic vaporizing inhaler mouthpiece, and the user may inhale the unvaporized liquid.

Electronic vaporizing inhalers are also known for providing inconsistent doses between draws. The aforementioned leaking is one cause of inconsistent doses because the membrane may be oversaturated or undersaturated near the vaporizer. If the membrane is oversaturated, then the user may experience a stronger than desired dose of vapor, and if the membrane is undersaturated, then the user may experience a weaker than desired dose of vapor. Additionally, small changes in the strength of the user's draw may provide stronger or weaker doses. Inconsistent dosing, along with leaking, can lead to faster consumption of the vaping liquid.

Additionally, conventional electronic vaporizing inhalers tend to rely on inducing high temperatures of a metal heating component configured to heat a liquid in the e-cigarette, thus vaporizing the liquid that can be breathed in. Problems with conventional electronic vaporizing inhalers may include the possibility of burning metal and subsequent breathing in of the metal along with the burnt liquid. In addition, some may not prefer the burnt smell caused by the heated liquid.

Electronic vaporizing inhalers are generally designed so that the liquid nicotine reservoir is arranged away from the metal heating component to prevent heating the unused liquid in the reservoir. This arrangement makes the inhaler device cumbersome and more complex to produce.

In electronic vaporizing inhalers, the vapor chamber is generally distant from the liquid reservoir and the mouthpiece to prevent heating of the liquid or the vapor before the user's draw.

Thus, a need exists in the art for an improved ultrasonic mist inhaler device which addresses at least some of the problems described herein.

### BRIEF SUMMARY

According to one aspect of the present invention, there is provided an ultrasonic mist inhaler device comprising: a sonication chamber; an ultrasonic transducer having an atomization surface which is provided within the sonication chamber, the ultrasonic transducer being configured to vibrate the atomization surface to atomize a liquid within the sonication chamber; an airflow delivery member which is coupled to the sonication chamber, the airflow delivery member being configured to deliver air into the sonication chamber along an airflow axis which intersects the atomization surface; and a mist outlet arrangement which provides a mist flow path along which a mist of air and atomized liquid can flow out from the sonication chamber to the exterior of the device.

In some embodiments, the airflow axis intersects the atomization surface substantially through the center of the atomization surface.

In some embodiments, the atomization surface of the ultrasonic transducer is substantially planar and the airflow axis is substantially perpendicular to the plane of the atomization surface.

In some embodiments, the device further comprises: a device housing which is elongate and which houses at least part of the sonication chamber, the ultrasonic transducer and the airflow delivery member, wherein the atomization surface of the ultrasonic transducer is substantially planar and the plane of the atomization surface is substantially parallel with the longitudinal length of the device housing.

In some embodiments, the device further comprises: an airflow arrangement which provides an air flow path from the exterior of the device to the airflow delivery member, wherein the airflow arrangement is configured to change the direction of a flow of air along the air flow path from a first direction to a second direction, the second direction being the direction of the airflow axis along which the airflow delivery member delivers air into the sonication chamber.

In some embodiments, the second direction is substantially perpendicular to the first direction.

In some embodiments, the device housing comprises: a side vent aperture which is provided in a side wall of the device housing, wherein the side vent aperture is in fluid communication with the airflow arrangement to provide an air flow path from the exterior of the side wall of the device to the airflow delivery member.

In some embodiments, the mist outlet arrangement is carried by a first end of the device housing and wherein the device housing comprises an end vent aperture which is provided at a second end of the device housing which is remote from the first end of the device housing.

In some embodiments, the device housing comprises a divider wall which divides the housing into: a first portion which houses at least part of the sonication chamber, the ultrasonic transducer and the airflow delivery member; and a second portion which is configured to house at least part of a liquid reservoir structure comprising a liquid chamber adapted to receive liquid to be atomized, wherein the divider wall is provided with an internal airflow aperture which allows air to flow along the air flow path from the second portion of the housing to the first portion of the housing.

In some embodiments, the mist outlet arrangement is carried by a first end of the device housing and wherein the device housing comprises a liquid reservoir structure coupling arrangement which is provided at a second end of the device housing which is remote from the first end of the device housing, the liquid reservoir structure coupling arrangement being configured to couple the housing to a liquid reservoir structure comprising a liquid chamber adapted to receive liquid to be atomized, wherein the device housing further comprises an airflow inlet aperture which is provided at the second end of the device housing, the airflow inlet aperture being in fluid communication with the airflow arrangement to provide an air flow path from the second end of the device housing to the airflow delivery member.

In some embodiments, the device further comprises: a liquid reservoir structure comprising: a liquid chamber adapted to receive liquid to be atomized; a further airflow arrangement which provides a further air flow path through part of the liquid reservoir structure; and an airflow outlet aperture which is in fluid communication with the further air flow path, the airflow outlet aperture being provided at a location on the liquid reservoir structure such that the airflow outlet aperture is at least partly aligned with the airflow inlet aperture of the device housing when the liquid reservoir structure is coupled to the device housing to provide a continuous air flow path from the exterior of the device, through part of the liquid reservoir structure and through the airflow arrangement to the airflow delivery member.

The arrangement of the means of ultrasonic vibrations disposed close to or at the bottom surface of the liquid chamber renders the fluid passage to the means of ultrasonic vibrations by gravity faster.

The arrangement of the means of ultrasonic vibrations disposed close to or at the top surface of the liquid chamber renders the mist passage to a mouthpiece shorter.

The expression "means of ultrasonic vibrations" is similar to the expression "ultrasonic oscillation component" used in the patent application PCT/IB2019/055192.

In the ultrasonic mist inhaler, in the arrangement close to or at the top surface, the means of ultrasonic vibrations may have at least an atomization surface parallel to the top surface.

In the ultrasonic mist inhaler, in the arrangement close to or at the top surface, the means of ultrasonic vibrations may have at least an atomization surface perpendicular to the top surface.

The atomization surface perpendicular to the top surface permits smaller thickness of the inhaler.

In the configuration of the atomization surface perpendicular to the top surface, the inhaler may comprise one of the following features taken alone or in combination:
- the liquid reservoir structure may comprise an inner container and an outer container wherein the inner container is surrounded by the outer container, the inner container forms the liquid reservoir,
- the capillary element is wrapped around the means of ultrasonic vibrations,
- the means of ultrasonic vibrations is supported by an elastic member,
- the elastic member is formed from an annular plate-shaped rubber,
- the elastic member has an inner hole wherein a groove is designed for maintaining the means of ultrasonic vibrations,
- the inner container has an opening into which the elastic member is introduced at least partly, the elastic member having a radial groove wherein the capillary element passes and penetrates the liquid chamber,
- the elastic member is inserted into the opening of the inner container by tight adjustment.

It is noted that the expression "mist" used in the invention means the liquid is not heated as usually in traditional inhalers known from the prior art. In fact, traditional inhalers use heating elements to heat the liquid above its boiling temperature to produce a vapor, which is different from a mist.

In fact, when sonicating liquids at high intensities, the sound waves that propagate into the liquid media result in alternating high-pressure (compression) and low-pressure (rarefaction) cycles, at different rates depending on the frequency. During the low-pressure cycle, high-intensity ultrasonic waves create small vacuum bubbles or voids in the liquid. When the bubbles attain a volume at which they can no longer absorb energy, they collapse violently during a high-pressure cycle. This phenomenon is termed cavitation. During the implosion very high pressures are reached locally. At cavitation, broken capillary waves are generated, and tiny droplets break the surface tension of the liquid and are quickly released into the air, taking mist form.

The ultrasonic mist inhaler according to the invention, wherein said liquid to be received in the liquid chamber comprises 57-70 % (w/w) vegetable glycerin and 30-43% (w/w) propylene glycol, said propylene glycol including nicotine and flavorings.

An ultrasonic mist inhaler or a personal ultrasonic atomizer device, comprising:
- a liquid reservoir structure comprising a liquid chamber or cartridge adapted to receive liquid to be atomized,
- a sonication chamber in fluid communication with the liquid chamber or cartridge,
wherein said liquid to be received in the liquid chamber comprises 57-70 % (w/w) vegetable glycerin and 30-43% (w/w) propylene glycol, said propylene glycol including nicotine and flavorings.

### BRIEF DESCRIPTION OF THE DRAWINGS

Some embodiments are illustrated by way of example and not limitation in the figures of the accompanying drawings.
FIG. 1 is an exploded view of components of the ultrasonic mist inhaler according to a first arrangement of means of ultrasonic vibrations disposed close to or at a bottom surface of a liquid chamber.
FIG. 2 is an exploded view of components of the inhaler liquid reservoir structure according to the first arrangement.
FIG. 3 is a cross section view of components of the inhaler liquid reservoir structure according to figure 1 or 2 wherein means of ultrasonic vibrations are arranged according to a first arrangement.
FIG. 4A is an isometric view of an airflow member of the inhaler liquid reservoir structure according to figures 2 and 3.
FIG. 4B is a cross section view of the airflow member shown in figure 4A.
FIG. 5 is a cross section view of a second arrangement of means of ultrasonic vibrations disposed close to or at a top surface of the liquid chamber.
FIG. 6 is a cross section view of a second arrangement of means of ultrasonic vibrations disposed close to or at the top surface of the liquid chamber.
FIG. 7 is a diagrammatic perspective view of an ultrasonic mist inhaler device of some embodiments.
FIG. 8 is a diagrammatic view of a liquid reservoir structure for use with the mist inhaler device of some embodiments.
FIG. 9 is a diagrammatic perspective view of part of a housing of an ultrasonic mist inhaler device of some embodiments.

### DETAILED DESCRIPTION

The foregoing summary, as well as the following detailed description of certain embodiments of the present invention, will be better understood when read in conjunction with the appended drawings.

As used herein, an element recited in the singular and preceded with the word "a" or "an" should be understood as not excluding plural of said elements, unless such exclusion is explicitly stated. Furthermore, the references to "one embodiment" of the present invention are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property.

The present invention is directed to an ultrasonic mist inhaler. The description of the invention and accompanying figures will be directed to the electronic vaporizing inhaler embodiment; however, other embodiments are envisioned, such as an inhaler for hookah, flavored liquids, medicine, and herbal supplements. Additionally, the device can be packaged to look like an object other than a cigarette. For instance, the device could resemble another smoking instrument, such as a pipe, water pipe, or slide, or the device could resemble another non-smoking related object.

Ultrasonic mist inhalers are either disposable or reusable. The term "reusable" as used herein implies that the energy storage device is rechargeable or replaceable or that the liquid is able to be replenished either through refilling or through replacement of the liquid reservoir structure. Alternatively, in some embodiments reusable electronic device is both rechargeable and the liquid can be replenished. A disposable embodiment will be described first, followed by a description of a reusable embodiment.

Conventional electronic vaporizing inhaler tend to rely on inducing high temperatures of a metal component configured to heat a liquid in the inhaler, thus vaporizing the liquid that can be breathed in. The liquid typically contains nicotine and flavorings blended into a solution of propylene glycol (PG) and vegetable glycerin (VG), which is vaporized via a heating component at high temperatures. Problems with conventional inhaler may include the possibility of burning metal and subsequent breathing in of the metal along with the burnt liquid. In addition, some may not prefer the burnt smell or taste caused by the heated liquid.

In contrast, aspects of the present disclosure include an ultrasonic mist inhaler that atomizes the liquid through ultrasonic vibrations, which produces micro bubbles in the liquid. When the bubbles come into contact with ambient air, droplets of about 0.25 to 0.5 microns spray into the air, thereby generating micro-droplets that can be absorbed through breathing, similar to breathing in a mist.

No heating elements are involved, thereby leading to no burnt elements and reducing second-hand smoke effects.

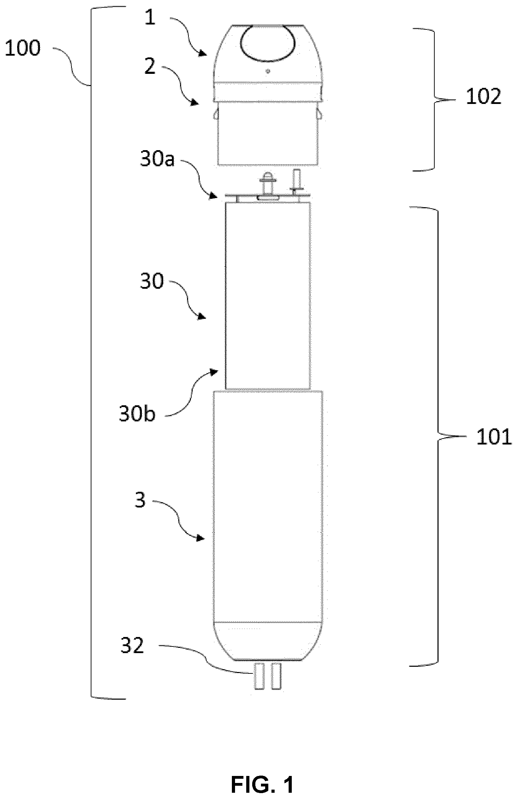

FIG. 1 to FIG. 4 illustrate a first arrangement of means of ultrasonic vibrations disposed close to or at a bottom surface of a liquid chamber.

FIG. 1 depicts a disposable ultrasonic mist inhaler 100 of the invention. As can be seen in FIG. 1, the ultrasonic mist inhaler 100 has a cylindrical body with a relatively long length as compared to the diameter. In terms of shape and appearance, the ultrasonic mist inhaler 100 is designed to mimic the look of a typical cigarette. For instance, the inhaler can feature a first portion 101 that primarily simulates the tobacco rod portion of a cigarette and a second portion 102 that primarily simulates a filter. In the disposable device, the first portion and second portion are regions of a single, but-separable device. The designation of a first portion 101 and a second portion 102 is used to conveniently differentiate the components that are primarily contained in each portion.

As can be seen in FIG. 1, the ultrasonic mist inhaler comprises a mouthpiece 1, a liquid reservoir structure 2 and a casing 3. The first portion 101 comprises the casing 3 and the second portion 102 comprises the mouthpiece 1 and the reservoir structure 2.

The first portion 101 contains the power supply energy.

An electrical storage device 30 powers the ultrasonic mist inhaler 100. The electrical storage device 30 can be a battery, including but not limited to a lithium-ion, alkaline, zinc-carbon, nickel-metal hydride, or nickel-cadmium battery; a super capacitor; or a combination thereof. In the disposable device, the electrical storage device 30 is not rechargeable, but, in the reusable device, the electrical storage device 30 would be selected for its ability to recharge. In the disposable device, the electrical storage device 30 is primarily selected to deliver a constant voltage over the life of the inhaler 100. Otherwise, the performance of the inhaler would degrade over time. Preferred electrical storage devices that are able to provide a consistent voltage output over the life of the device include lithium-ion and lithium polymer batteries.

The electrical storage device 30 has a first end 30a that generally corresponds to a positive terminal and a second end 30b that generally corresponds to a negative terminal. The negative terminal is extending to the first end 30a.

Because the electrical storage device 30 is located in the first portion 101 and the liquid reservoir structure 2 is located in the second portion 102, the joint needs to provide electrical communication between those components. In the present invention, electrical communication is established using at least an electrode or probe that is compressed together when the first portion 101 is tightened into the second portion 102.

In order for this device to be reusable, the electrical storage device 30 is rechargeable. The casing 3 contains a charging port 32.

The integrated circuit 4 has a proximal end 4a and a distal end 4b. The positive terminal at the first end 30a of the electrical storage device 30 is in electrical communication with a positive lead of the flexible integrated circuit 4. The negative terminal at the second end 30b of the electrical storage device 30 is in electrical communication with a negative lead of the integrated circuit 4. The distal end 4b of the integrated circuit 4 comprise a microprocessor. The microprocessor is configured to process data from a sensor, to control a light, to direct current flow to means of ultrasonic vibrations 5 in the second portion 102, and to terminate current flow after a preprogrammed amount of time.

The sensor detects when the ultrasonic mist inhaler 100 is in use (when the user draws on the inhaler) and activates the microprocessor. The sensor can be selected to detect changes in pressure, air flow, or vibration. Preferably, the sensor is a pressure sensor. In the digital device, the sensor takes continuous readings which in turn requires the digital sensor to continuously draw current, but the amount is small and overall battery life would be negligibly affected.

Additionally, the integrated circuit 4 may comprise a H bridge, preferably formed by 4 MOSFETs to convert a direct current into an alternate current at high frequency.

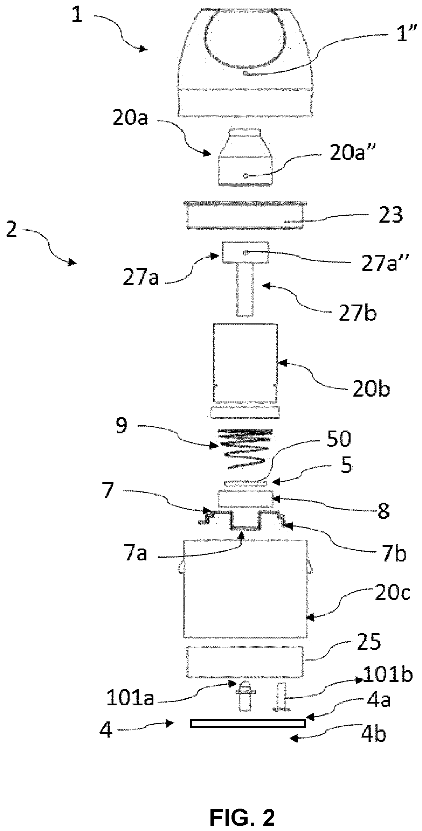

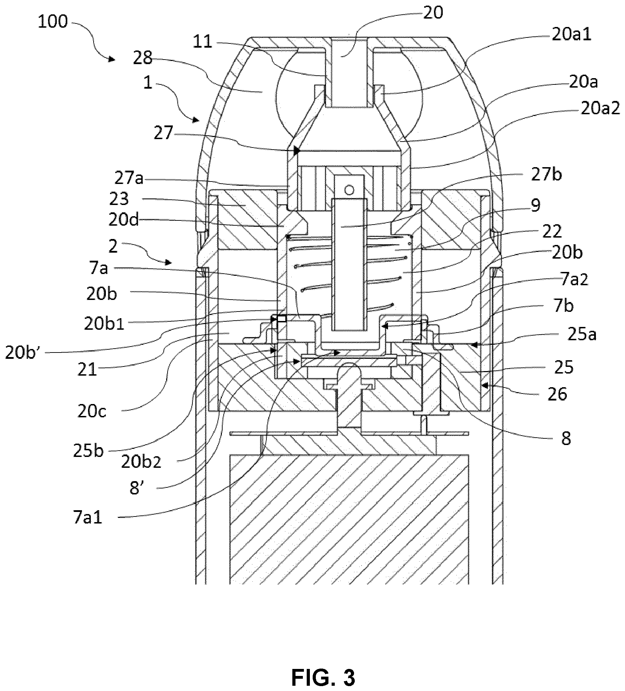

Referring to FIG. 2 and FIG. 3, illustrations of a liquid reservoir structure 2 are shown. The liquid reservoir structure 2 comprises a liquid chamber 21 adapted to receive liquid to be atomized and a sonication chamber 22 in fluid communication with the liquid chamber 21.

In this arrangement, the liquid reservoir structure 2 comprises an inhalation channel 20 providing an air passage from the sonication chamber 22 toward the surroundings.

As an example of sensor position, the sensor may be located in the sonication chamber 22.

The inhalation channel 20 has a frustoconical element 20a and an inner container 20b.

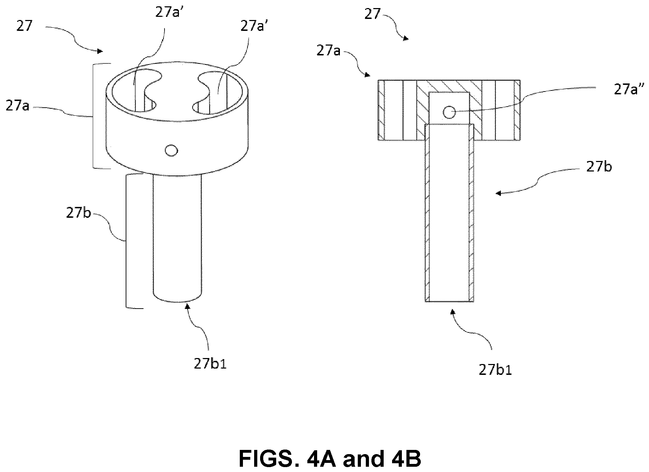

As depicted in Figure 4A and 4B, further the inhalation channel 20 has an airflow member 27 for providing air flow from the surroundings to the sonication chamber 22.

The airflow member 27 has an airflow bridge 27a and an airflow duct 27b made in one piece, the airflow bridge 27a having two airway openings 27a' forming a portion of the inhalation channel 20 and the airflow duct 27b extending in the sonication chamber 22 from the airflow bridge 27a for providing the air flow from the surroundings to the sonication chamber.

The airflow bridge 27a cooperates with the frustoconical element 20a at the second diameter 20a2.

The airflow bridge 27a has two opposite peripheral openings 27a" providing air flow to the airflow duct 27b.

The cooperation with the airflow bridge 27a and the frustoconical element 20a is arranged so that the two opposite peripheral openings 27a" cooperate with complementary openings 20a" in the frustoconical element 20a.

The mouthpiece 1 and the frustoconical element 20a are radially spaced and an airflow chamber 28 is arranged between them.

As depicted in FIG. 1 and 2, the mouthpiece 1 has two opposite peripheral openings 1".

The peripheral openings 27a", 20a", 1" of the airflow bridge 27a, the frustoconical element 20a and the mouthpiece 1 directly supply maximum air flow to the sonication chamber 22.

The frustoconical element 20a includes an internal passage, aligned in the similar direction as the inhalation channel 20, having a first diameter 20a1 less than that of a second diameter 20a2, such that the internal passage reduces in diameter over the frustoconical element 20a.

The frustoconical element 20a is positioned in alignment with the means of ultrasonic vibrations 5 and a capillary element 7, wherein the first diameter 20a1 is linked to an inner duct 11 of the mouthpiece 1 and the second diameter 20a2 is linked to the inner container 20b.

The inner container 20b has an inner wall delimiting the sonication chamber 22 and the liquid chamber 21.

The liquid reservoir structure 2 has an outer container 20c delimiting the outer wall of the liquid chamber 21.

The inner container 20b and the outer container 20c are respectively the inner wall and the outer wall of the liquid chamber 21.

The liquid reservoir structure 2 is arranged between the mouthpiece 1 and the casing 3 and is detachable from the mouthpiece 1 and the casing 3.

The liquid reservoir structure 2 and the mouthpiece 1 or the casing 3 may include complimentary arrangements for engaging with one another; further such complimentary arrangements may include one of the following: a bayonet type arrangement; a threaded engaged type arrangement; a magnetic arrangement; or a friction fit arrangement; wherein the liquid reservoir structure 2 includes a portion of the arrangement and the mouthpiece 1 or the casing 3 includes the complimentary portion of the arrangement.

In the reusable device, the components are substantially the same. The differences in the reusable device vis-a-vis the disposable device are the accommodations made to replace the liquid reservoir structure 2.

As shown in FIG. 3, the liquid chamber 21 has a top wall 23 and a bottom wall 25 closing the inner container 20b and the outer container 20c of the liquid chamber 21.

The capillary element 7 is arranged between a first section 20b1 and a second section 20b2 of the inner container 20b.

The capillary element 7 has a flat shape extending from the sonication chamber to the liquid chamber.

As depicted in FIG. 2 or 3, the capillary element 7 comprises a central portion 7a in U-shape and a peripheral portion 7b in L-shape.

The L-shape portion 7b extends into the liquid chamber 21 on the inner container 20b and along the bottom wall 25.

The U-shape portion 7a is contained into the sonication chamber 21. The U-shape portion 7a on the inner container 20b and along the bottom wall 25.

In the ultrasonic mist inhaler, the U-shape portion 7a has an inner portion 7a1 and an outer portion 7a2, the inner portion 7a1 being in surface contact with an atomization surface 50 of the means of ultrasonic vibrations 5 and the outer portion 7a2 being not in surface contact with the means of ultrasonic vibrations 5.

The bottom wall 25 of the liquid chamber 21 is a bottom plate 25 closing the liquid chamber 21 and the sonication chamber 22. The bottom plate 25 is sealed, thus preventing leakage of liquid from the sonication chamber 22 to the casing 3.

The bottom plate 25 has an upper surface 25a having a recess 25b on which is inserted an elastic member 8. The means of ultrasonic vibrations 5 are supported by the elastic member 8. The elastic member 8 is formed from an annular plate-shaped rubber having an inner hole 8' wherein a groove is designed for maintaining the means of ultrasonic vibrations 5.

The top wall 23 of the liquid chamber 21 is a cap 23 closing the liquid chamber 21.

The top wall 23 has a top surface 23 representing the maximum level of the liquid that the liquid chamber 21 may contain and the bottom surface 25 representing the minimum level of the liquid in the liquid chamber 21.

The top wall 23 is sealed, thus preventing leakage of liquid from the liquid chamber 21 to the mouthpiece 1.

The top wall 23 and the bottom wall 25 are fixed to the liquid reservoir structure 2 by means of fixation such as screws, glue, or friction.

As depicted in FIG. 3, the elastic member 8 is in line contact with the means of ultrasonic vibrations 5 and prevents contact between the means of ultrasonic vibrations 5 and the inhaler walls, suppression of vibrations of the liquid reservoir structure are more effectively prevented. Thus, fine particles of the liquid atomized by the atomizing member can be sprayed farther.

As depicted in FIG. 3, the inner container 20b has openings 20b' between the first section 20b1 and the second section 20b2 from which the capillary element 7 is extending from the sonication chamber 21. The capillary element 7 absorbs liquid from the liquid chamber 21 through the apertures 20b'. The capillary element 7 is a wick. The capillary element 7 transports liquid to the sonication chamber 22 via capillary action. Preferably the capillary element 7 is made of bamboo fibers; however, cotton, paper, or other fiber strands could be used for a wick material.

In one arrangement of the ultrasonic mist inhaler 100, as can be seen in FIG. 3, the means of ultrasonic vibrations 5 are disposed directly below the capillary element 7.

The means of ultrasonic vibrations 5 may be a transducer. For example, the means of ultrasonic vibrations 5 may be a piezoelectric transducer, preferably designed in a circular plate-shape. The material of the piezoelectric transducer is preferably in ceramic.

A variety of transducer materials can also be used for the means of ultrasonic vibrations 5.

The end of the airflow duct 27b1 faces the means of ultrasonic vibrations 5. The means of ultrasonic vibrations 5 are in electrical communication with electrical contactors 101a, 101b. It is noted that, the distal end 4b of the integrated circuit 4 has an inner electrode and an outer electrode. The inner electrode contacts the first electrical contact 101a which is a spring contact probe, and the outer electrode contacts the second electrical contact 101b which is a side pin. Via the integrated circuit 4, the first electrical contact 101a is in electrical communication with the positive terminal of the electrical storage device 30 by way of the microprocessor, while the second electrical contact 101b is in electrical communication with the negative terminal of the electrical storage device 30.

The electrical contacts 101a, 101b crossed the bottom plate 25. The bottom plate 25 is designed to be received inside the perimeter wall 26 of the liquid reservoir structure 2. The bottom plate 25 rests on complementary ridges, thereby creating the liquid chamber 21 and sonication chamber 22.

The inner container 20b comprises a circular inner slot 20d on which a mechanical spring is applied.

By pushing the central portion 7a1 onto the means of ultrasonic vibrations 5, the mechanical spring 9 ensures a contact surface between them.

The liquid reservoir structure 2 and the bottom plate 25 can be made using a variety of thermoplastic materials.

When the user draws on the ultrasonic mist inhaler 100, an air flow is drawn from the peripheral openings 1" and penetrates the airflow chamber 28, passes the peripheral openings 27a" of the airflow bridge 27a and the frustoconical element 20a and flows down into the sonication chamber 22 via the airflow duct 27b directly onto the capillary element 7. At the same time, the liquid is drawn from the reservoir chamber 21 by capillarity, through the plurality of apertures 20b', and into the capillary element 7. The capillary element 7 brings the liquid into contact with the means of ultrasonic vibrations 5 of the inhaler 100. The user's draw also causes the pressure sensor to activate the integrated circuit 4, which directs current to the means of ultrasonic vibrations 5. Thus, when the user draws on the mouthpiece 1 of the inhaler 100, two actions happen at the same time. Firstly, the sensor activates the integrated circuit 4, which triggers the means of ultrasonic vibrations 5 to begin vibrating. Secondly, the draw reduces the pressure outside the reservoir chamber 21 such that flow of the liquid through the apertures 20b' begins, which saturates the capillary element 7. The capillary element 7 transports the liquid to the means of ultrasonic vibrations 5, which causes bubbles to form in a capillary channel by the means of ultrasonic vibrations 5 and mist the liquid. Then, the mist liquid is drawn by the user.

The ultrasonic mist inhaler 100 of the present disclosures is a more powerful version of current portable medical nebulizers, in the shape and size of current e-cigarettes and with a particular structure for effective vaporization. It is a healthier alternative to cigarettes and current e-cigarettes products.

The ultrasonic mist inhaler 100 of the present disclosures has particular applicability for those who use electronic inhalers as a means to quit smoking and reduce their nicotine dependency. The ultrasonic mist inhaler 100 provides a way to gradually taper the dose of nicotine.

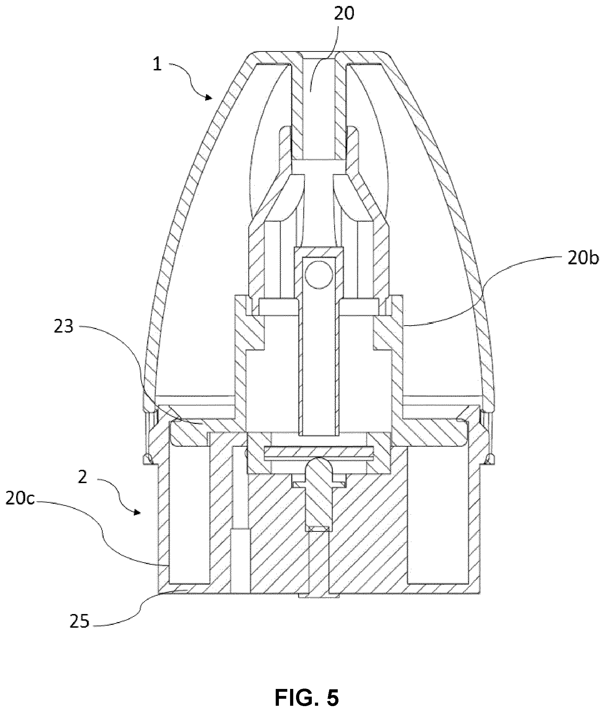

FIG. 5 illustrate a second arrangement of means of ultrasonic vibrations disposed close to or at a top surface of the liquid chamber.

The mouthpiece 1 is extending down to the bottom end of the inner container 20b. While the liquid chamber 21 is delimited by the outer container 20c and an inner peripheral container 20e joined by a bottom wall 25. The liquid chamber is closed by a top wall 23.

The top wall 23 has the top surface 23 representing the maximum level of the liquid that the liquid chamber 21 may contain and the bottom surface 25 representing the minimum level of the liquid in the liquid chamber 21. The top wall 23 is sealed, thus preventing leakage of liquid from the liquid chamber 21 to the mouthpiece 1.

As depicted in FIG. 5, the means of ultrasonic vibrations 5 has a flat shape having an atomization surface 50 parallel to the top surface 23 and disposed close to or at the top surface 23.

The top wall 23 is fixed to the liquid reservoir structure 2 by means of fixation such as screws or glue.

Contrary to the first arrangement, the capillary element 7 is not shown to make the illustrated features more clear.

Of course, the capillary element 7 may have the same configuration that the first arrangement. In this second arrangement, the L-shape is extending down to the bottom surface 25.

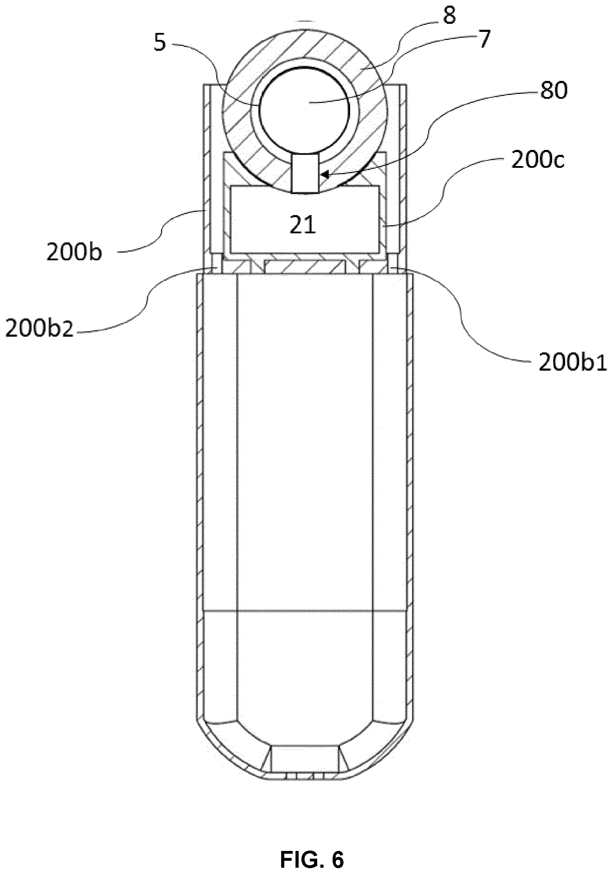

FIG. 6 illustrate a second arrangement of means of ultrasonic vibrations disposed close to or at a top surface of the liquid chamber.

In the second arrangement, the liquid reservoir structure 2 may comprise an inner container 200c and an outer container 200b wherein the inner container 200c is surrounded by the outer container 200b, the inner container 200c forms the liquid chamber 21.

The capillary element 7 is wrapped around the circular shape of the means of ultrasonic vibrations 5.

Further, the means of ultrasonic vibrations 5 is supported by an elastic member 8 formed from an annular plate-shaped rubber.

The elastic member 8 has an inner hole wherein a groove 80 is designed for maintaining the capillary element 7 and the means of ultrasonic vibrations 5.

The inner container 200c has a flat opening into which the elastic member 8 is introduced at least partly, the elastic member 8 having a radial groove 80 wherein the capillary element 7 passes and penetrates the liquid chamber.

The inner container 200c is sealed with the elastic member 8, for example, by glue or mastic disposed around the peripheral of the flat opening.

In this third arrangement, the means of electrical contacts rise through electrical contact holes 200b1 and 200b2 on either side of inner container 200c and connect with the rear of the means of ultrasonic vibrations 5.

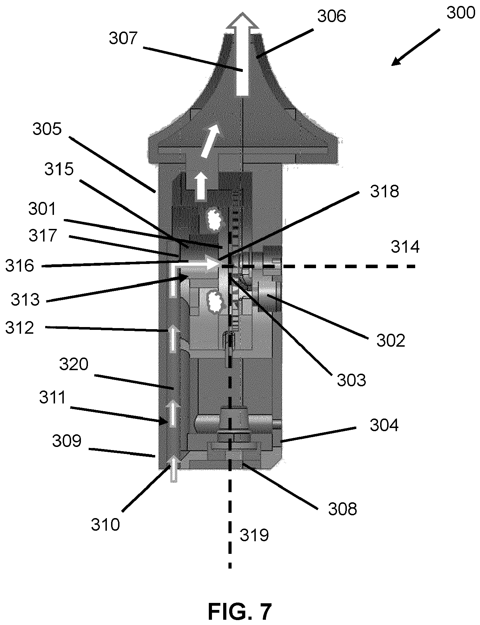

Referring now to FIG. 7 of the accompanying drawings, an ultrasonic mist inhaler device 300 of some embodiments comprises many of the same elements and functions in a similar manner to the arrangements described above. For simplicity, some of the elements are omitted from FIG. 7 but it is to be appreciated that this embodiment may comprise any or all of the elements of the arrangement described above in any combination.

The device 300 comprises a sonication chamber 301 and an ultrasonic transducer 302 having an atomization surface 303 which is provided within the sonication chamber 301. The ultrasonic transducer 302 is configured to vibrate the atomization surface 303 to atomize a liquid within the sonication chamber 301.

In this embodiment, the device 300 comprises a housing 304 which houses at least part of the sonication chamber 301 and the ultrasonic transducer 302, along with other components of the device 300. The housing 304 is an elongate housing. A first end 305 of the housing 304 carries a mist outlet arrangement 306. The mist outlet arrangement 306 provides a mist flow path 307 along which a mist of air and atomized liquid can flow out from the sonication chamber 301 to the exterior of the device 300.

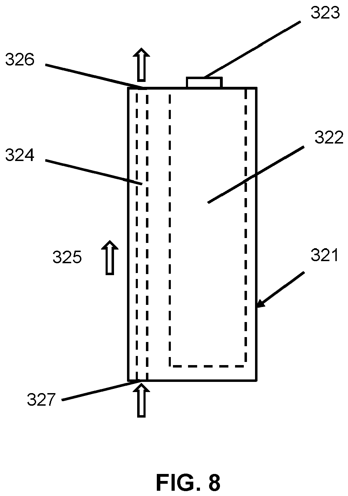

The device 300 comprises a liquid reservoir structure coupling arrangement 308 which is provided at a second end 309 of housing 304. The liquid reservoir structure coupling arrangement 308 is configured to couple the housing 304 to a liquid reservoir structure which comprises a liquid chamber adapted to receive liquid to be atomized. The liquid reservoir structure is shown in FIG. 8, which is discussed in more detail below.

The device 300 is provided with an airflow inlet aperture 310 at the second end 309 of the housing 304. The airflow inlet aperture 310 is in fluid communication with an airflow arrangement 311 which provides an airflow path 312 through the device 300.

The device 300 comprises an airflow delivery member 313 which is coupled to the sonication chamber 301 and configured to deliver air into the sonication chamber 301 along an air flow axis 314 which intersects the atomization surface 303.

The airflow delivery member 313 comprises a body portion 315 which is provided with an airflow passage 316. The airflow passage 316 provides an air flow path through the airflow delivery member 313 between a first aperture 317 and a second aperture 318. The second aperture 318 is superimposed on and spaced apart from the atomization surface 303 such that air flowing out from the aperture 318 flows along the air flow axis 314 which intersects the atomization surface 303.

In some embodiments, the air flow axis 314 intersects the atomization surface 303 substantially through the center of the atomization surface 303. In some embodiments, the atomization surface 303 of the ultrasonic transducer 302 is substantially planar or planar and aligned with a plane 319. In some embodiments, the air flow axis 314 is substantially perpendicular or perpendicular to the plane 319 or the atomization surface 303.

In this embodiment, the plane 319 of the atomization surface 303 is substantially parallel with the longitudinal length of the housing 304. The atomization surface 303 is thus vertical when the device 300 is standing upright on one end of its elongate housing 304.

The airflow arrangement 311 provides the air flow path 312 from the exterior of the device 300 to the airflow delivery member 313. The airflow arrangement 311 comprises a first portion 320 which provides a first portion of the air flow path 312 for air to flow in a first direction. The first air flow direction is substantially aligned or aligned with the longitudinal length of the device 300. The airflow arrangement 311 is configured to change the direction of the flow of air along the flow path 312 from the first direction to a second direction. The second direction being the direction of the air flow axis 314 along which the airflow delivery member 313 delivers air into the sonication chamber 301. In some embodiments, the second direction is substantially perpendicular or perpendicular to the first direction.

Referring now to FIG. 8 of the accompanying drawings, the device 300 of some embodiments comprises a liquid reservoir structure 321 which comprises a liquid chamber 322 adapted to receive liquid to be atomized. The liquid reservoir structure 321 comprises a coupling arrangement 323 which is configured to couple or releasably couple to the liquid reservoir structure coupling arrangement 308 provided on the housing 304. The coupling arrangement is configured to allow liquid to be conveyed, for instance under capillary action, from the liquid chamber 322 to the sonication chamber 301 as described above.

The liquid reservoir structure 321 comprises a further air flow arrangement 324 which provides a further air flow path 325 through part of the liquid reservoir structure 321. The liquid reservoir structure 321 further comprises an air flow outlet aperture 326 which is in fluid communication with the further airflow path 325. The air flow outlet aperture 326 is provided at a location on the liquid reservoir structure 321 such that the air flow outlet aperture 326 is at least partly aligned with the air flow inlet aperture 310 of the housing 304 when the liquid reservoir structure 321 is coupled to the housing 304. The liquid reservoir structure 321 comprises an air inlet aperture 327 which is in fluid communication with the further airflow arrangement 324. When the liquid reservoir structure 321 is coupled to the housing 304, a continuous air flow path is provided from the exterior of the device 300, through the further airflow arrangement 324 of the liquid reservoir structure 321, through the airflow arrangement 311 to the airflow delivery member 313.

In use, a user draws on the device 300 by inhaling and creating a negative air pressure at the mist outlet arrangement 306. The negative air pressure draws air through the airflow arrangement and the further airflow arrangement so that air flow along the air flow paths through the liquid reservoir structure 321 and the housing 304. The airflow arrangement 311 changes the direction of the air flow and aligns the air flow along the air flow axis 314. The airflow delivery member 313 delivers air into the sonication chamber 304 along the air flow axis 314 which intersects the atomization surface 303 such that the air flow acts in a direction towards the atomization surface 303 and against a capillary element carrying liquid which is superimposed on the atomization surface 303, as described above. The force of the flow of air acting against the capillary element and the atomization surface 303 helps to improve the efficiency of the sonication of liquid within the sonication chamber 301 by exerting a biasing force on the liquid which biases the capillary element and the liquid against the atomization surface 303. The ultrasonic transducer 302 vibrates the atomization surface 303 which atomizes the liquid into a mist. The mist of air and the atomized liquid flow out from the sonication chamber 301 along the mist flow path 307, through the mist outlet arrangement 306 and into the mouth and lungs of the user.

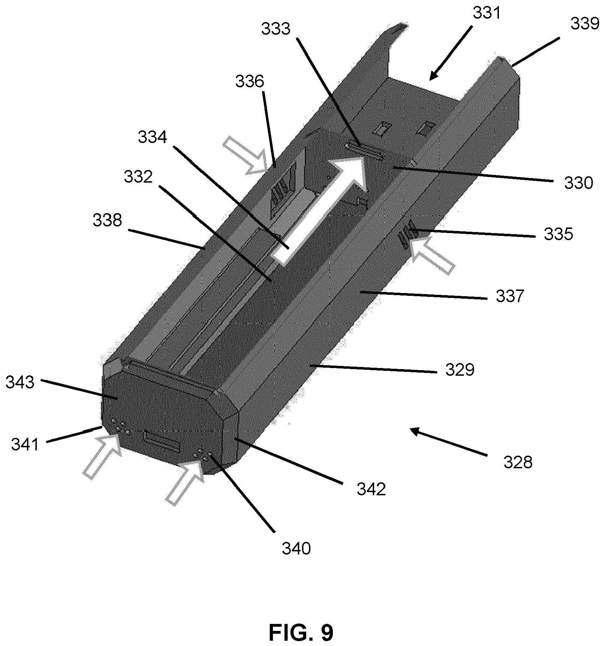

Referring now to FIG. 9 of the accompanying drawings, an ultrasonic mist inhaler device 328 of some embodiments comprises many of the same elements as the device 300 shown in FIG. 7. However, the device 328 of the embodiment comprises a modified housing 329.

The housing 329 of this embodiment is elongate and houses at least part of the sonication chamber, the ultrasonic transducer and the airflow delivery member in the same configuration as the device 300 described above. In this embodiment, the atomization surface of the ultrasonic transducer is also substantially planar and the plane of the atomization surface is substantially parallel with the longitudinal length of the housing 329.

The housing 329 of the device 328 comprises a divider wall 330 which divides the housing 329 into a first portion 331 which houses at least part of the sonication chamber, the ultrasonic transducer and the airflow delivery member, and a second portion 332 which is configured to house at least part of a liquid reservoir structure (not shown) comprising a liquid chamber adapted to receive liquid to be atomized. In this embodiment, the liquid reservoir structure is preferably a removable structure which is configured to be inserted in and at least partly housed within the housing 329. In some embodiments, the liquid reservoir structure is a disposable pod which may be pre-filled with liquid to be atomized of configured to be filled by such a liquid.

In this embodiment, the divider wall 330 is provided with an internal airflow aperture 333 which allows air to flow along an air flow path 334 from the second portion 332 of the housing 329 to the first portion 331 of the housing 329.

In some embodiments, the housing 329 comprises at least one side vent aperture 335, 336 which is provided in a side wall 337, 338 of the housing 329. In this embodiment, the housing 329 is provided with two side vent apertures 335, 336, with one side vent aperture 335, 336 being provided on each side wall 337, 338 of the housing 329. Each side vent aperture 335, 336 is in fluid communication with the airflow arrangement of the device 328 to provide an air flow path from the exterior of the side walls 337, 338 of the device 328 to the airflow delivery member, as indicated generally by the arrows in FIG. 9.

In this embodiment, the mist outlet arrangement is carried by a first end 339 of the housing 329 and the housing 329 comprises at least one end vent aperture 340, 341 which is provided at a second end 342 of the housing 329 which is remote from the first end 339 of the housing 329. In this embodiment, each end vent aperture 340, 341 comprises a plurality of spaced apart apertures. In this embodiment, there are two spaced apart vent apertures 340, 341 which are provided at respective sides of a base 343 of the device 328. It is, however, to be appreciated that other embodiments comprise a greater or fewer number of vent apertures than the number shown in FIG.9. In some embodiments, the cross-section of the vent apertures 340, 341 and the side vent apertures 335, 336 is sized to allow ample air to balance such that the negative pressure required to take a puff resembles a traditional cigarette.

Other embodiments of the invented ultrasonic mist inhaler are easily envisioned, including medicinal delivery devices.

It is to be understood that the above description is intended to be illustrative, and not restrictive. For example, the above-described embodiments may be used in combination with each other. In addition, many modifications may be made to adapt a particular situation or material to the teachings of the invention without departing from its scope.

When used in this specification and claims, the terms "comprises" and "comprising" and variations thereof mean that the specified features, steps or integers are included. The terms are not to be interpreted to exclude the presence of other features, steps or components.

## Claims

1. An ultrasonic mist inhaler device comprising:
a sonication chamber;
an ultrasonic transducer having an atomization surface which is provided within the sonication chamber, the ultrasonic transducer being configured to vibrate the atomization surface to atomize a liquid within the sonication chamber;
an airflow delivery member which is coupled to the sonication chamber, the airflow delivery member being configured to deliver air into the sonication chamber along an airflow axis which intersects the atomization surface; and
a mist outlet arrangement which provides a mist flow path along which a mist of air and atomized liquid can flow out from the sonication chamber to the exterior of the device.

2. The device of claim 1, wherein the airflow axis intersects the atomization surface substantially through the center of the atomization surface.

3. The device of claim 1 or claim 2, wherein the atomization surface of the ultrasonic transducer is substantially planar and the airflow axis is substantially perpendicular to the plane of the atomization surface.

4. The device of any one of the preceding claims, wherein the device further comprises:
a device housing which is elongate and which houses at least part of the sonication chamber, the ultrasonic transducer and the airflow delivery member, wherein the atomization surface of the ultrasonic transducer is substantially planar and the plane of the atomization surface is substantially parallel with the longitudinal length of the device housing.

5. The device of any one of the preceding claims, wherein the device further comprises:
an airflow arrangement which provides an air flow path from the exterior of the device to the airflow delivery member, wherein the airflow arrangement is configured to change the direction of a flow of air along the air flow path from a first direction to a second direction, the second direction being the direction of the airflow axis along which the airflow delivery member delivers air into the sonication chamber.

6. The device of claim 5, wherein the second direction is substantially perpendicular to the first direction.

7. The device of claim 5 or claim 6 as dependent on claim 4, wherein the device housing comprises:
a side vent aperture which is provided in a side wall of the device housing, wherein the side vent aperture is in fluid communication with the airflow arrangement to provide an air flow path from the exterior of the side wall of the device to the airflow delivery member.

8. The device of any one of claims 5 to 7 as dependent on claim 4, wherein the mist outlet arrangement is carried by a first end of the device housing and wherein the device housing comprises an end vent aperture which is provided at a second end of the device housing which is remote from the first end of the device housing.

9. The device of any one of claims 5 to 8, wherein the device housing comprises a divider wall which divides the housing into:
a first portion which houses at least part of the sonication chamber, the ultrasonic transducer and the airflow delivery member; and
a second portion which is configured to house at least part of a liquid reservoir structure comprising a liquid chamber adapted to receive liquid to be atomized, wherein the divider wall is provided with an internal airflow aperture which allows air to flow along the air flow path from the second portion of the housing to the first portion of the housing.

10. The device of claim 5 or claim 6 as dependent on claim 4, wherein the mist outlet arrangement is carried by a first end of the device housing and wherein the device housing comprises a liquid reservoir structure coupling arrangement which is provided at a second end of the device housing which is remote from the first end of the device housing, the liquid reservoir structure coupling arrangement being configured to couple the housing to a liquid reservoir structure comprising a liquid chamber adapted to receive liquid to be atomized, wherein the device housing further comprises an airflow inlet aperture which is provided at the second end of the device housing, the airflow inlet aperture being in fluid communication with the airflow arrangement to provide an air flow path from the second end of the device housing to the airflow delivery member.

11. The device of claim 10, wherein the device further comprises:
a liquid reservoir structure comprising:
a liquid chamber adapted to receive liquid to be atomized;
a further airflow arrangement which provides a further air flow path through part of the liquid reservoir structure; and
an airflow outlet aperture which is in fluid communication with the further air flow path, the airflow outlet aperture being provided at a location on the liquid reservoir structure such that the airflow outlet aperture is at least partly aligned with the airflow inlet aperture of the device housing when the liquid reservoir structure is coupled to the device housing to provide a continuous air flow path from the exterior of the device, through part of the liquid reservoir structure and through the airflow arrangement to the airflow delivery member.
